# FASCICULE DE BREVET EUROPEEN

(11) **EP 1 411 780 B1**
(45) Date de publication et mention de la délivrance du brevet: **09.07.2008**
(21) Numéro de dépôt: 02774854.0
(22) Date de dépôt: 29.07.2002
(51) Int. Cl.: A23F 5/44

(54) **CHICOREE TORREFIEE SOLUBLE A HAUTE TENEUR INULINE**
LÖSLICHE GERÖSTETE ZICHORIE MIT HOHEM INULINGEHALT
SOLUBLE ROASTED CHICORY WITH HIGH INULIN CONTENT

(30) Priorité: 30.07.2001 FR 0110202
(43) Date de publication de la demande: 28.04.2004
(73) Titulaire: FINALER, 59310 Orchies (FR)
(72) Inventeur: JANSSENS, Myriam, F-59152 Chereng (FR)
(74) Mandataire: Hennion, Jean-Claude
(86) Numéro de dépôt international: PCT/FR2002/002716
(87) Numéro de publication internationale: WO 2003/011042

(56) Documents cités:
- EP-A- 0 824 109
- WO-A-96/24256
- FR-A- 2 245 293
- PORDAB ZOFIA: "Some physico-chemical and technological aspects of the production of soluble chicory. Part II." PRACE INST. LAB. BAD. PRZEM. SPOZYW., vol. 35, 1981, pages 123-130, XP002197477
- R. CLARKE: "Coffee Vol. 5: Related beverages" 1987 , ELSEVIER APPLIED SCIENCE , LONDON XP002220987 page 46 -page 53; tableau 10 page 38; tableau 5
- JADWIGA CIESLAK: "Some physico-chemical and technological aspects of the production of soluble chicory. Part I." PRACE INST. LAB. BAD. PRZEM. SPOZYW., vol. 35, 1981, pages 113-122, XP002197478
- PAZOLA Z ET AL: "Changes in carbohydrates during the production of coffee substitute extracts, especially in the roasting process." FOOD CHEMISTRY 1979 INST. OF HUMAN NUTR., AGRIC. ACAD., MAZOWIECKA 48, 60-623 POZNAN, POLAND, vol. 4, no. 1, pages 41-52, XP008010271
- DATABASE FSTA [en ligne] INTERNATIONAL FOOD INFORMATION SERVICE (IFIS), FRANFURT/MAIN, DE; GULYAEV V N ET AL: "Use of aluminium tubes for packaging soluble chicory in paste form." Database accession no. 79-3-06-f0274 XP002197479 & KONSERVNAYA I OVOSHCHESUSHIL'NAYA PROMYSHLENNOST' 1978 VSES. NAUCHNO-ISSLED. INST. KONSERVNOI PROMYSHLENNOSTI I SPETSIAL'NOI PISHCHEVOI TEKHNOLOGII, USSR, no. 6, 1978, pages 26-29,
- DATABASE FSTA [en ligne] INTERNATIONAL FOOD INFORMATION SERVICE (IFIS), FRANFURT/MAIN, DE; WESTERDIJK C E: "Chicory (Cichorium intybus L. var. Sativus) for inulin production." Database accession no. 97-1-07-j0149 XP002197481 & AGRO FOOD INDUSTRY HI-TECH, vol. 8, no. 1, 1997, pages 5-6, Lelystad, Netherlands

## Description

La présente invention concerne l'industrie agro-alimentaire, s'agissant de chicorée torréfiée soluble utilisable comme ingrédient alimentaire dans l'alimentation humaine voire animale.

A travers les travaux de GIBSON GR et de ROBERFROID MB est apparu le concept des ingrédients alimentaires dits prébiotiques. Il s'agit d'ingrédients non digestibles qui stimulent sélectivement la croissance et /ou l'activité d'une bactérie spécifique ou d'un nombre restreint d'espèces bactériennes du colon, favorisant ainsi la santé du consommateur. Pour être prébiotique, l'ingrédient alimentaire doit remplir les conditions suivantes . Il ne doit pas être hydrolysé ni être absorbé dans la partie supérieure du tractus digestif. Il doit être un substrat sélectif pour les bactéries spécifiques ou un nombre restreint de bactéries favorables du colon, dont le développement et/ou le métabolisme est stimulé. Il doit être capable par conséquent de modifier favorablement la composition de la microflore du colon. Enfin il doit induire des effets systémiques stimulant la santé du consommateur.

A ce jour, les seuls ingrédients alimentaires qui ont été reconnus et utilisés comme remplissant les conditions ci-dessus, sont des fructanes ou oligofructoses. En référence à l'article de GIBSON et ROBERFROID de 1995, le terme de fructo oligosaccharide (FOS) désigne des oligofructoses ayant un degré de polymérisation de 9 au maximum et le terme inuline désigne un oligofructose ayant un degré de polymérisation plus élevé, compris entre 10 et 60, en moyenne de 12.

Cependant, dans la littérature on utilise fréquemment le terme générique inuline pour désigner à la fois l'inuline proprement dite et les fructo oligosaccharides (FOS). C'est ce terme générique inuline qui sera adopté dans le présent texte.

Ces hydrates de carbone ne sont pas digestibles dans la partie supérieur du tractus ; ils ne subissent pas l'action enzymatique entre autre des amylases, saccharases, maltases.... Ainsi, l'inuline et les FOS passent de l'iléon vers le colon sans être absorbés et sont considérés à ce titre comme des fibres alimentaires. Cependant, bien qu'ils ne soient pas métabolisés par les enzymes digestives, ils ont quand même une valeur calorique. La microflore intestinale peut les métaboliser en acides gras à courtes chaînes (acides organiques aliphatiques) et en lactate (acide organique). La valeur calorique de l'inuline et des fructo oligosaccharides (FOS) est comprise entre 4,2 et 6,3 kj/g soit entre 1 et 1,5 kcal/g.

Par ailleurs l'effet bifidostimulant a été démontré par des expérimentations tant in vitro qu'in vivo pour les fructo oligosaccharides et pour l'inuline. En particulier une expérimentation faite au département des sciences biomédicales de la Faculté Agricole de Tokyo a permis de montrer une amélioration de la micro flore fécale après l'administration de 8 g de fructo oligosaccharides pendant deux semaines. Le nombre des bifidobactéries dans les selles a été multiplié par dix. Le pH moyen de selles a baissé de 0,3 et les auteurs ont constaté une amélioration du métabolisme lipidique. Ainsi a été démontré que les ingrédients dits prébiotiques changent efficacement la composition de la microflore intestinale , de sorte que les bactéries favorables prédominent sur les espèces potentiellement dangereuses.

On sait que l'inuline est l'un des composants de la chicorée, pouvant représenter de l'ordre de 70 % de la matière sèche de la racine de chicorée. Cette racine est d'ailleurs utilisée pour l'extraction de l'inuline. Par contre en ce qui concerne l'utilisation de la chicorée comme ingrédient alimentaire , le procédé conventionnel comporte plusieurs étapes de déshydratation, de torréfaction, d'extraction et d'atomisation au cours desquelles la teneur en inuline diminue jusqu'à notamment sous l'action conjuguée de la température et de l'humidité. C'est ce qui permet d'obtenir le goût, l'arôme, la flaveur, la couleur particulières de la chicorée torréfiée soluble conventionnelle qui est utilisée comme ingrédient alimentaire.

Selon le demandeur, la chicorée torréfiée soluble obtenue par le procédé conventionnel - dénommée ci-après chicorée conventionnelle - aurait une teneur en inuline de l'ordre de 18-19% et en tout cas inférieure à 20%.

L'objet de la présente invention est de proposer une chicorée naturelle torréfiée soluble, en poudre, comportant une haute teneur en inuline et fructo oligosaccharide comprise entre 50 et 60 % en poids par rapport à la matière sèche, telle que définie dans la présente revendication 1.

Grâce à une telle teneur, la consommation d'un bol d'une préparation traditionnelle à base de cette chicorée, à haute teneur en inuline, apporte au consommateur de l'ordre de 10% des fibres alimentaires qui lui sont nécessaires dans une journée.

C'est un autre objet de l'invention que de proposer un procédé de production d'une chicorée naturelle torréfiée soluble en poudre qui présente une haute teneur en Inuline. De manière conventionnelle ce procédé comporte les étapes suivantes :
a) déshydratation des racines fraîches pour obtenir des cossettes,
b) torréfaction des cossettes,
c) refroidissement et concassage des cossettes torréfiées,
d) extraction de la chicorée par passage d'eau chaude sur les cossettes torréfiées et concassées et
e) atomisation de l'extrait de chicorée.

De manière caractéristique selon l'invention, l'opération de torréfaction est réalisée à une température de l'ordre de 130°C et avec un taux d'humidité réduit dans le torréfacteur.

Il est à noter que la température de torréfaction, selon le procédé conventionnel est de l'ordre de 145°C. Ainsi, selon l'invention, on choisit de travailler avec une température anormalement basse, conjuguée avec un taux d'humidité réduit, de manière à éviter les réactions qui se produisent habituellement à savoir la rupture de la chaîne glucidique et la production de sucres libres. Certes la torréfaction conventionnelle a pour effet de conférer à la chicorée sa saveur particulière qui résulte de l'amertume initiale de la chicorée, de la production des sucres libres et de la condensation des sucres entre eux ou avec des acides aminés. Le fait que , selon l'invention, il y ait moins de sucres libres produits, moins de condensation des sucres entre eux ou avec des acides aminés et moins de dégradation des principes amers (lactones sesquiterpeniques) entraîne un accroissement de l'amertume par rapport à la chicorée conventionnelle. Cependant cette amertume n'est pas rédhibitoire, certains consommateurs pouvant l'accepter spontanément compte-tenu du bienfait apporté par la présence des fibres alimentaires en grandes quantités. De plus cette amertume peut facilement être masquée dans des mélanges de la chicorée à haute teneur en inuline, selon l'invention, avec d'autres composants, que ce soit du café, du chocolat ou autre composant alimentaire habituel.

Le taux d'humidité réduit dans le torréfacteur est obtenu par deux voies qui de préférence se combinent. Selon la première voie, on ne procède à aucune addition d'eau dans le torréfacteur, y compris pendant la phase communément appelée « le piquet » qui est la phase essentielle durant laquelle les cossettes finissent de cuire, et au cours de laquelle la boule du torréfacteur continue de tourner pendant environ vingt minutes après la première étape de chauffage progressif jusqu'à atteindre une température de l'ordre de 130°C.

Selon la seconde voie, le taux d'humidité est réduit dans le torréfacteur par échappement de la vapeur d'eau produite.

La teneur en inuline de la chicorée de l'invention peut également dépendre, en partie, d'une part de la sélection variétale et d'autre part du choix du terroir. S'agissant de la sélection variétale, il convient de retenir parmi les semences possibles les variétés dont le potentiel en terme de rendement, de teneur en matière sèche et de teneur en inuline est élevé. Plus le pourcentage de matière sèche soluble est élevé et plus est élevé, non seulement le pourcentage d'inuline, mais également le degré de polymérisation de cette inuline. On retient plus particulièrement comme références des variétés dénommées respectivement Orchies et Turquoise, produites par la société Florimond Desprez et on choisit de préférence des racines fraîches qui proviennent d'une variété végétale dont le potentiel en terme de rendement, de teneur en matière sèche et de teneur en inuline est équivalent ou supérieur au potentiel desdites références.

S'agissant du terroir, il est préférable de réaliser un semi précoce en vue de respecter la longueur des chaînes d'inuline et pour cela de choisir un sol sablonneux ou sablo-limoneux. Le climat favorable est celui des zones maritimes c'est-à-dire doux et humide.

La chicorée naturelle torréfiée soluble , en poudre, qui est obtenue par le procédé précité se distingue également par certaines caractéristiques.

Certes on a déjà proposé dans le document US.5.958.497 une chicorée soluble en poudre et qui comporte une teneur en inuline en poids qui est comprise entre 30 et 65%. Cependant dans ce document le but visé est d'éliminer les inconvénients du procédé conventionnel de production de chicorée mettant en oeuvre les étapes de torréfaction, d'extraction et de séchage, dont il est dit qu'ils consistent dans des propriétés sensorielles non satisfaisantes et dans un excès d'hygroscopicité. La recherche d'une teneur en inuline importante a pour but de diminuer l'hygroscopicité de la chicorée obtenue. Le procédé mis en oeuvre dans ce document ne correspond absolument plus aux étapes conventionnelles de production de chicorée, puisqu'en particulier on ne retrouve plus l'étape de torréfaction. Selon ce nouveau procédé, on commence par réaliser un extrait de chicorée par l'une ou l'autre des méthodes d'extraction à savoir extraction par l'eau d'une chicorée étuvée ou extraction par pressage des racines de chicorée. Cet extrait de chicorée est chauffé dans un réacteur tubulaire en vue d'hydrolyser une partie de l'inuline contenue dans l'extrait pour augmenter la teneur en sucres réducteurs de l'extrait. On sèche ensuite l'extrait provenant de l'extracteur pour obtenir une poudre. On passe la poudre à travers un extrudeur, celui-ci étant chauffé en sorte que la poudre soit traitée pendant une durée de 5 mn à une température comprise entre 180 et 250°C en vue d'obtenir en sortie d'extrudeur un produit caramélisé. On refroidit ce produit caramélisé puis on réalise un broyage pour obtenir la chicorée soluble moulue.

Le produit obtenu se distingue par sa composition qui comprend une teneur en inuline en poids entre 40 et 65% et de plus une teneur en sucres réducteurs entre 4 et 6% , une combinaison de fructose et de glucose inférieure à 5% ; de plus cette poudre de chicorée soluble a un indice de couleur qui est compris entre 1,0 et 2,5, fourchette de valeurs qui englobe les indices habituellement obtenus pour la chicorée conventionnelle (de 1,5 à plus de 2).

Comparativement la chicorée naturellement riche en inuline obtenue par le procédé de l'invention a un indice de couleur, mesuré dans les mêmes conditions que celui du document US.5.958.497, qui est très nettement inférieur puisqu'il est compris entre 0,4 et 0,7. En ce qui concerne les sucres réducteurs, leur teneur est en moyenne supérieure à 10%.

En ce qui concerne la combinaison de fructose et de glucose, la teneur est supérieure à 5%, étant notamment comprise entre 7 et 9%.

Contrairement à ce qui est décrit dans le document US.5.958.497, le procédé de l'invention met en oeuvre les mêmes étapes et le même matériel que dans le procédé conventionnel de production d'une chicorée soluble utilisable comme ingrédient alimentaire. C'est le mérite du demandeur que d'avoir aménagé les conditions opératoires du procédé conventionnel dans le but d'obtenir une chicorée torréfiée soluble ayant une teneur élevée en inuline, teneur comprise entre 50 et 60 % qui s'avère suffisante pour pouvoir justifier auprès du consommateur un apport bienfaisant de fibres alimentaires avec tous les avantages que la présence de telles fibres apporte dans l'équilibre alimentaire du consommateur, notamment le transit intestinal. L'enseignement de ce document US.5.958.497 ne peut à l'évidence conduire au produit et au procédé de l'invention puisque d'une part il s'oppose à la mise en oeuvre du procédé conventionnel incluant la torréfaction, conduisant donc à un produit qui n'est pas torréfié et d'autre part la présence d'inuline est recherchée pour éviter le caractère hydroscopique de la poudre soluble. Les racines fraîches qui ont servi à la production de la chicorée à haute teneur en inuline qui va être décrite ci-après proviennent de semences de la variété Orchies.

Les racines fraîches ont tout d'abord été déshydratées pour l'obtention des cossettes. Les cossettes ont été introduites dans un torréfacteur chargé à environ 1000 kg et adapté de manière à permettre l'évacuation de la vapeur. Le torréfacteur est soumis à un chauffage indirect, progressif , grâce à un brûleur au gaz , jusqu'à une température de l'ordre de 130°C. Lorsque cette température est atteinte , l'alimentation en gaz est coupée. Le temps de piquet est de l'ordre de 25 à 35mn, c'est-à-dire le temps pendant lequel la boule du torréfacteur continue de tourner, permettant que la cuisson des cossettes se termine. A l'issue de cette phase, les cossettes torréfiées sont vidangées dans un refroidisseur, étant soumises pendant une durée déterminée à une circulation d'air pulsé. Après refroidissement, les cossettes torréfiées sont concassées. Après concassage, intervient la phase d'extraction. Durant cette phase, on fait circuler de l'eau à plus de 80°C à contre-courant sur les grains de chicorée concassée. L'extrait de chicorée, c'est-à-dire le jus récupéré, est filtré, centrifugé avant d'être atomisé.

La poudre de chicorée, naturelle, torréfiée et soluble qui a été ainsi obtenue contient 98% en poids de matière sèche. De plus en poids par rapport à ladite matière sèche, elle contient 59,1% d'inuline, 4,3% de saccharose, 7,3% de fructose libre et de l'ordre de 0,5% de glucose libre.

En ce qui concerne l'indice de couleur, obtenu grâce à une mesure d'absorbance à 500 nm d'une solution à 1% de chicorée, la valeur obtenue varie de 0,598 à 0,634.

De plus la teneur en sucre réducteur est de 10,3g/100g de chicorée (dosage par la méthode Bertrand, résultats exprimés en glucose).

Comparativement une poudre de chicorée naturelle, torréfiée et soluble obtenue par le procédé conventionnel contient en moyenne et en poids par rapport à la matière sèche : 18,7% d'inuline, 2,4 % de saccharose, 22,5% de fructose libre et 3,1 % de glucose libre. De plus son indice de couleur, obtenu dans les mêmes conditions, a une valeur qui varie de 1,567 à 2,005. Ainsi la chicorée naturelle torréfiée soluble, en poudre, de la présente invention présente des caractéristiques en terme de composition et d'indice de couleur se distinguant nettement de celles de la chicorée conventionnelle, comme de celles de la chicorée décrite dans le brevet US.5.958.497.

## Revendications

1. Chicorée naturelle torréfiée soluble, en poudre, présentant une haute teneur en inuline et fructo-oligosaccharides comprise entre 50 et 60 % en poids par rapport à la matière sèche, **caractérisée en ce que** l'indice de couleur, mesuré par absorption à 500 nm d'une solution à 1 % de ladite chicorée, est compris entre 0,4 et 0,7 et **en ce que** sa teneur combinée en fructose et glucose est supérieure à 5%.

2. Chicorée selon la revendication 1 **caractérisée en ce que** sa teneur combinée en fructose et glucose est comprise entre 7 % et 9 % en poids par rapport à la matière sèche.

3. Procédé de production d'une chicorée naturelle torréfiée et soluble en poudre, à haute teneur en inuline et fructo oligosaccharides (FOS), comprenant les étapes suivantes :
a) déshydratation des racines fraîches pour obtenir des cossettes,
b) torréfaction des cossettes,
c) refroidissement et concassage des cossettes torréfiées,
d) extraction de la chicorée par passage d'eau chaude sur les cossettes torréfiées et concassées, et
e) atomisation de l'extrait de chicorée,
**caractérisé en ce que** l'opération de torréfaction est réalisée à une température de l'ordre de 130°C et avec un taux d'humidité réduit dans le torréfacteur.

4. Procédé selon la revendication 3 **caractérisé par** l'absence d'apport d'eau lors de la torréfaction.

5. Procédé selon l'une des revendications 3 ou 4 **caractérisé par** une libre évacuation de la vapeur d'eau produite lors de l'opération de torréfaction.

6. Procédé selon l'une des revendications 3 à 5 **caractérisé en ce que** les racines fraîches proviennent d'une variété végétale dont le potentiel en terme de rendement, de teneur en matière sèche et de teneur en inuline est équivalent ou supérieur au potentiel des variétés dénommées ORCHIES ou TURQUOISE de la société FLORIMOND DEPREZ.

## Claims

1. Soluble roasted natural chicory in powder form, presenting a high content of inulin and fructo oligosaccharides (FOS) lying in the range 50% to 60% by weight relative to the dry matter, **characterised in that** the colour index measured by the absorbance of a 1% solution of said chicory at 500 nm lies in the range 0.4 to 0.7 and **in that** its combined content of fructose plus glucose is greater than 5%.

2. Chicory according to claim 1, **characterised in that** its combined content of fructose plus glucose lies in the range 7% to 9% by weight relative to the dry matter.

3. A method of producing roasted and soluble natural chicory in powder form having high content of inulin and fructo oligosaccharides (FOS), the method comprising the following steps:
a) dehydrating fresh roots to obtain cossettes;
b) roasting the cossettes;
c) cooling and pounding the roasted cossettes;
d) extracting chicory by passing hot water over the roasted and pounded cossettes; and
e) atomizing the chicory extract;
the method being **characterized in that** the roasting is performed at a temperature of about 130°C with a reduced moisture content in the roaster.

4. A method according to claim 3, **characterized by** the absence of water being supplied during roasting.

5. A method according to claim 3 or claim 4, **characterized by** free removal of the water vapor produced during roasting.

6. A method according to one of claims 3 to 5, **characterized in that** the fresh roots come from a plant variety whose potential in terms of yield, dry matter content, and inulin content is equivalent to or greater than the potential of the varieties named Orchies or Turquoise from the supplier Florimond Desprez.

## Patentansprüche

1. Natürliche, geröstete, lösliche Zichorie als Pulver, welche einen hohen Gehalt an Inulin und Fructooligosacchariden aufweist, der zwischen 50 und 60 Gew.-% im Verhältnis zur Trockenmasse liegt, **dadurch gekennzeichnet, daß** der Farbindex, gemessen durch Absorption bei 500 nm, von einer einprozentigen Lösung dieser Zichorie zwischen 0,4 und 0,7 liegt und daß ihr kombinierter Gehalt an Fructose und Glucose größer als 5% ist.

2. Zichorie nach Anspruch 1, **dadurch gekennzeichnet, daß** ihr kombinierter Gehalt an Fructose und Glucose zwischen 7 und 9 Gew.-% im Verhältnis zur Trockenmasse liegt.

3. Verfahren zur Herstellung einer natürlichen, gerösteten und löslichen Zichorie als Pulver mit hohem Gehalt an Inulin und Fructooligosacchariden (FOS), umfassend die folgenden Schritte:
a) Dehydratisierung der frischen Wurzeln, um Schnitzel zu erhalten,
b) Rösten der Schnitzel,
c) Kühlen und Zerkleinern der gerösteten Schnitzel,
d) Extraktion der Zichorie durch Durchlauf von warmem Wasser über die gerösteten und zerkleinerten Schnitzel und
e) Zerstäuben des Zichorienextraktes,
**dadurch gekennzeichnet, daß** der Röstvorgang bei einer Temperatur in der Größenordnung von 130°C mit einem verminderten Feuchtigkeitsgrad im Röster ausgeführt wird.

4. Verfahren nach Anspruch 3, **gekennzeichnet durch** die Abwesenheit einer Wasserzuführung bei dem Rösten.

5. Verfahren nach einem der Ansprüche 3 oder 4, **gekennzeichnet durch** ein ungehindertes Abziehen des Wasserdampfs, der bei dem Röstvorgang erzeugt wird.

6. Verfahren nach einem der Ansprüche 3 bis 5, **dadurch gekennzeichnet, daß** die frischen Wurzeln von einer Pflanzenart kommen, deren Potential hinsichtlich Ausbeute, Trockenmassegehalt und Inulingehalt äquivalent oder über dem Potential der Arten ist, die von der Gesellschaft FLORIMOND DEPREZ als ORCHIES oder TURQUOISE bezeichnet werden.
